# EUROPÄISCHE PATENTSCHRIFT

(11) **EP 3 005 962 B1**
(45) Veröffentlichungstag und Bekanntmachung des Hinweises auf die Patenterteilung: **22.05.2019**
(21) Anmeldenummer: 15186068.1
(22) Anmeldetag: 21.09.2015
(51) Int. Cl.: A61B 17/70, A61B 90/00

(54) **SICHERUNGSHÜLSE FÜR EINE PEDIKELSCHRAUBE**
SECURING SLEEVE FOR A PEDICLE SCREW
DOUILLE DE SECURITE POUR UNE VIS PEDICULAIRE

(30) Priorität: 07.10.2014 DE 102014114530
(43) Veröffentlichungstag der Anmeldung: 13.04.2016
(73) Patentinhaber: Aesculap AG, 78532 Tuttlingen (DE)
(72) Erfinder: Gnoth, Barbara, 78194 Immendingen (DE); Ricci, Denis, 78532 Tuttlingen (DE); Ackermann, Janina, 78532 Tuttlingen (DE)
(74) Vertreter: Winter, Brandl, Fürniss, Hübner, Röss, Kaiser, Polte - Partnerschaft mbB

(56) Entgegenhaltungen:
- EP-A1- 2 777 573
- US-A1- 2008 077 135
- US-A1- 2012 323 278
- US-A1- 2013 096 635

## Beschreibung

Die vorliegende Erfindung betrifft eine Sicherungshülse zum Aufnehmen und Sichern der verlängerten Flanken bzw. Abbrechflanken einer Pedikelschraube.

### Hintergrund der Erfindung

In der minimalinvasiven Wirbelsäulenchirurgie bieten Pedikelschrauben mit verlängerten Flanken besonders bei der Wirbelreposition die Möglichkeit, auf diverse Instrumente verzichten zu können und führen somit zu einem deutlich kleineren Hautschnitt. Dadurch läßt sich die Belastung des Patienten während der Operation reduzieren und die Heilung nach der Operation beschleunigen. Bei der Wirbelreposition werden zuerst Tulpenkopf-Pedikelschauben mit verlängerten Flanken in die Wirbelpedikel eingeschraubt, wonach zum Ausrichten der Pedikelschrauben zueinander ein Ausrichtestab in die Tulpenköpfe mehrere Pedikelschrauben eingeführt wird. Sind die Pedikelschrauben an den gewünschten Positionen in die Wirbelpedikel eingebracht und korrekt ausgerichtet, so bricht der Chirurg die verlängerten Flanken der Pedikelschrauben bzw. deren Tulpenköpfe ab. Um dies zu erleichtern und um ein gratloses Abbrechen der Flanken zu ermöglichen, weisen Pedikelschrauben mit verlängerten Flanken zwischen dem Schraubenkopf und den Flanken jeweils eine Sollbruchstelle auf.

Durch die verlängerten Flanken erhöht sich die Belastung auf die Sollbruchstelle in Folge von Verformungskräften, welche durch Gewebespannungen und die Handhabung des Chirurgen verursacht werden. Daher muss, um ein vorzeitiges Abbrechen der Flanken zu verhindern, die Position der Flanken gesichert werden.

Hierfür sind Sicherungsringe bzw. -hülsen bekannt, die über die verlängerten Flanken einer Pedikelschraube geschoben werden können, um die Flanken während des Einbringens und Ausrichtens der Pedikelschraube zu stabilisieren.

### Stand der Technik

Eine simple Flankenschutzhülse in Form eines einfachen Rings, welcher über die Flanken einer Pedikelschraube gestreift werden kann, ist beispielsweise aus der DE102011111403A1 bekannt. Der innere Radius dieser Flankenschutzhülse entspricht dem äußeren Radius des Tulpenkopfes der Pedikelschraube und die innere Umfangsoberfläche der Flankenschutzhülse ist glatt ausgebildet. Nachteilig an dieser Lösung ist, dass die Position der Flanken innerhalb der Flankenschutzhülse nicht fest definiert ist, da sich die Flanken entlang der glatten inneren Umfangsoberfläche der Flankenschutzhülse unkontrollierbar bewegen können. Auch die Aufnahmetiefe der Flanken in die Flankschutzhülse ist nicht definiert und die Flankenschutzhülse kann entlang der Axialrichtung der Flanken unerwünscht verrutschen.

Zudem erfüllt die Flankenschutzhülse keine weitere Funktion neben der Stabilisierung der Flanken, somit erfolgt eine Ausrichtung mehrerer Pedikelschrauben mit jeweils einer solchen Flankenschutzhülse durch Einführen eines Korrekturstabs in die jeweiligen Tulpenköpfe der Pedikelschrauben. Dadurch befindet sich der Korrekturstab sehr nahe an dem Gewebe des Patienten und durch etwaiges Verformen des Korrekturstabs durch den Chirurgen kann das Gewebe unnötig beschädigt werden.

Eine besser definierte Positionierung der Flanken in dem Sicherungsring bieten ein aus dem Stand der Technik allgemein bekannter Sicherungsring, sowie insbesondere der aus der US20130096635A1 bekannte Sicherungsring.

Der allgemein bekannte Sicherungsring ist ein Halbring, der zwei Aufnahmeöffnungen/separate Kanäle für die zwei Flanken einer Pedikelschraube aufweist. Auch dieser Sicherungsring kann über die Flanken einer Pedikelschraube gestreift werden, allerdings ist das Einfädeln der Flanken in die jeweilige Aufnahmeöffnung zeitaufwändig und schwierig. Auch bei dem allgemein bekannten Sicherungsring ist die Aufnahmetiefe der Flanken in die Flankschutzhülse nicht definiert und der Sicherungsring kann entlang der Axialrichtung der Flanken unerwünscht verrutschen. Abgesehen von der Stabilisierung der Flanken erfüllt der allgemein bekannte Ring keine weiteren Funktionen und die vorstehend beschriebene Problematik des Einführens eines Korrekturstabs in die Tulpenköpfe der Pedikelschrauben bei der Ausrichtung dieser zueinander bleibt bestehen. Zudem verengt sich, da die Aufnahmeöffnungen die Flanken vollständig umschließen, der durch den die Flanken definierte instrumentenzugängliche Durchgang zwischen den Flanken. Dies erschwert dem Chirurgen die Positionierung der Pedikelschrauben, da der Zugangskanal zwischen den Flanken durch den Ring verengt wird.

Der Sicherungsring der US20130096635A1 weist ebenfalls zwei Aufnahmeöffnungen für die zwei Flanken einer Pedikelschraube auf. Um die Flanken hierbei in Position zu halten und ein unerwünschtes Bewegen der Flanken zu verhindern, umschließen die Aufnahmeöffnungen die Flanken sehr eng. Somit gestaltet sich auch hier ein Einfädeln der Flanken in die jeweilige Aufnahmeöffnung als zeitaufwändig und schwierig.

An seinem der Pedikelschraube abgewandten Ende weist der Sicherungsring in jeder der Aufnahmeöffnungen jeweils ein Stoppteil auf, welches die Aufnahmetiefe der Flanken in den Sicherungsring begrenzt und definiert. Um ein unerwünschtes Abstreifen der Sicherungshülse von den Flanken zu verhindern, weist jede Aufnahmeöffnung zudem eine enge Nut auf, in die ein entsprechender Nutenstein einer verlängerten Flanke eingesetzt wird, wenn die Flanke in die Aufnahmeöffnung eingeführt wird. Zum Lösen dieser Verankerung und Entfernen des Sicherungsrings von den Flanken der Pedikelschraube weist der Sicherungsring an seiner Außenseite jeweils neben einer Nut einen elastisch verformbaren Druckarm auf, auf den der Chirurg von außen Druck ausüben kann, wodurch sich der Druckarm in die Nut bewegt, den Nutenstein aus der Nut drückt und die Flanke somit von dem Sicherungsring trennt.

Zwar bietet dieser Sicherungsring eine sichere und definierte Positionierung der Flanken einer Pedikelschraube in dem Sicherungsring, allerdings führt der Aufbau des Sicherungsrings mit den mehreren Stoppteilen, engen Aufnahmeöffnungen, engen Nuten und Druckarmen zu vielen Hinterschneidungen und toten Winkeln, in denen sich Verschmutzungen bzw. bei der Operation anfallende Gewebeteilchen festsetzen können. Die Reinigung eines Sicherungsrings mit solch kompliziertem Aufbau ist sehr zeitaufwändig und schwierig.

Abgesehen von der Stabilisierung der Flanken erfüllt der aus der US20130096635A1 bekannte Sicherungsring keine weiteren Funktionen und die Problematik der Gewebebeschädigung während des Einführens eines Korrekturstabs in die Tulpenköpfe der Pedikelschrauben bei der Ausrichtung dieser zueinander bleibt ebenfalls bestehen. Auch verringert sich, da die Aufnahmeöffnungen die Flanken zumindest teilweise umschließen, der Innendurchmesser des durch die Flanken definierten instrumentenzugänglichen Durchgangs zwischen den Flanken und dem Chirurgen wird die Positionierung der Pedikelschrauben erschwert.

Um die Problematik der Verringerung des Innendurchmessers des instrumentenzugänglichen Zugangs zwischen den Flanken zu umgehen, kann, wie beispielsweise aus der US8496661 B2 bekannt, ein Instrument selbst (z.B. ein Schraubendreher) mit einem Sicherungsring ausgestattet sein. Hierbei ist der Sicherungsring einstückig mit dem Schraubendreher ausgebildet, so dass sich die Flanken gleichzeitig mit und entsprechend der Drehung des Schraubendrehers bewegen. Somit wird eine Scherwirkung auf die verlängerten Flanken vermieden.

Nachteilig hierbei ist, dass das Instrument, welches einstückig mit dem Sicherungsring verbunden ist, nur für solche Schrauben verwendet werden kann, für die der Sicherungsring passt. Auch die Problematik der Gewebebeschädigung während des Einführens eines Korrekturstabs in die Tulpenköpfe der Pedikelschrauben bei der Ausrichtung dieser zueinander bleibt bestehen.

### Kurzbeschreibung der Erfindung

Gegenüber der US 20130096635A1 als nächstkommendem Stand der Technik liegt der vorliegenden Erfindung die Aufgabe zugrunde, eine Sicherungshülse zu schaffen, die eine definierte Positionierung der Flanken einer Pedikelschraube in der Sicherungshülse ermöglicht, ohne dabei den instrumentenzugänglichen Zugang zwischen den Flanken zu verengen und die zudem einfach zu reinigen ist.

Diese Aufgabe wird gelöst durch die Sicherungshülse mit den Merkmalen des Anspruchs 1. Vorteilhafte Weiterbildungen und Abwandlungen der Erfindung sind Gegenstand der Unteransprüche.

Der Kerngedanke der vorliegenden Erfindung besteht darin, die Sicherungshülse anstatt mit Aufnahmeöffnungen, welche die Flanken der Pedikelschraube jeweils zumindest teilweise umschließen, mit einer Anzahl an Nutensteinen/Vorsprüngen an der Innenseite der Sicherungshülse auszustatten, die dazu angepasst sind, um zwischen die Flanken einlegbar/einführbar zu sein und die Flanken in einem festen definierten Abstand zueinander zu halten. Da die Nutensteine nur zwischen die Flanken eingelegt/eingeführt werden, diese aber nicht umschließen, wird der Durchgang/Innenquerschnitt zwischen den Flanken nicht durch die Sicherungshülse verengt. Um die Aufnahmetiefe der Flanken in die Sicherungshülse zu definieren, weist die Sicherungshülse zudem vorzugsweise an ihrer Innenseite einen Anschlagsring bzw. Axialanschlag in Form eines radial einwärts ragenden Vorsprungs auf, der dazu vorgesehen ist, auf der Oberkante der Flanken (distale Stirnkante) aufzuliegen. Durch die einfache Ausgestaltung der Sicherungshülse mit dem Anschlagsring und der Anzahl an Nutensteinen lassen sich die Flanken einer Pedikelschraube sicher in einer definierten Axial- und/oder Drehposition in der Sicherungshülse fixieren, ohne dass viele tote Winkel oder Hinterschneidungen und somit hygienische Nachteile entstehen.

Um das Aufsetzen der Sicherungshülse auf die Flanken zu vereinfachen, verjüngt sich in einer Ausführungsform mindestens einer der Anzahl an Nutensteinen an der Innenseite der Sicherungshülse konisch d.h. vorzugsweise V-förmig entgegen der Einführungsrichtung der Flanken der Pedikelschraube in die Sicherungshülse. Die Nutensteine werden hierbei in den Schlitz/Spalt zwischen den Flanken der Pedikelschraube/Tulpenkopf eingesetzt. Durch die konische Form werden die Flanken maximal beabstandet zueinander gehalten.

Der Anschlagsring/Axialanschlag dient dazu, die Aufnahmetiefe der Flanken der Pedikelschraube in die Sicherungshülse axial zu begrenzen. Der Anschlagring/Axialanschlag ist ein vorzugsweise ringförmiger Vorsprung an der Innenseite der Sicherungshülse, der sicht entlang der gesamten Umfangslinie der Sicherungshülse oder eines Teils davon erstreckt. Vorzugsweise ist der Anschlagring/Axialanschlag nahe dem der Pedikelschraube fernen Ende der Sicherungshülse ausgebildet. Damit der zwischen den Flanken der Pedikelschraube gebildete Durchgang nicht durch den Anschlagsring/Axialanschlag verengt wird, ist in einer bevorzugten Ausführungsform die radiale Erstreckung des Anschlagsrings/Axialanschlags in den Innenumfang der Sicherungshülse von dem Innenumfang des durch die Flanken der Pedikelschraube definierten Durchgangs begrenzt. Das heißt, der Anschlagsring/Axialanschlag steht nicht über die Flanken in den zwischen den Flanken gebildeten Durchgang radial nach innen hervor und schränkt somit nicht das Einführen von Instrumenten in den Durchgang ein.

In einer weiteren bevorzugten Ausführungsform entspricht die radiale Erstreckung der Anzahl an Nutensteinen in den Innenumfang der Sicherungshülse maximal der radialen Erstreckung des Anschlagsrings/Axialanschlags in den Innenumfang der Sicherungshülse. Hierdurch wird eine Verengung des zwischen den Flanken gebildeten Durchgangs durch die Nutensteine verhindert.

Die Sicherungshülse kann weiterhin dadurch verbessert werden, dass an dem der Pedikelschraube fernen Ende der Sicherungshülse weiterhin mindestens eine Ausrichtenut/Doppelnut zur Queraufnahme eines Korrekturstabes zur Ausrichtung der Flanken mehrerer Pedikelschrauben zueinander ausgebildet ist. Dies ermöglicht es, dass die Sicherungshülse selbst zur Ausrichtung mehrerer Pedikelschrauben mittels eines Korrekturstabs verwendet werden kann. Hierbei wird der Korrekturstab in die Ausrichtenuten mehrerer Sicherungshülsen quer zur Hülsenachse eingeführt. Dies bedeutet, dass der Korrekturstab nicht unmittelbar neben dem Gewebe des Patienten zu liegen kommt, wie bei der aus dem Stand der Technik bekannten Ausrichtung der Pedikelschrauben durch Einführen eines Korrekturstabs in die Tulpenköpfe/zwischen die Flanken der Pedikelschrauben. Somit verringert sich das Risiko der Gewebeschädigung aufgrund eines Verbiegens des Korrekturstabs direkt neben dem Patientengewebe und die Ausrichtung wird weniger invasiv. Außerdem werden die Flanken weniger belastet (weil nicht gegeneinander aufgebogen) und damit deren Sollbruchstellen nicht gebrochen.

In einer vorteilhaften Ausführungsform fluchtet die mindestens eine Ausrichtenut an der Sicherungshülse mit mindestens einem der Nutensteine. Hierdurch wird eine Bewegung der Ausrichtenut durch den Korrekturstab beim Ausrichten der Pedikelschrauben optimal auf die Flanken der Pedikelschraube übertragen.

Gemäß einer vorteilhaften Weiterbildung weist die Sicherungshülse weiterhin mindestens ein Rückhaltelement an dem der Pedikelschraube nahen Ende der Sicherungshülse zur Verhinderung eines unerwünschten Abstreifens der Sicherungshülse von den Flanken entgegen und/oder in der Einführungsrichtung der Flanken in die Sicherungshülse auf. Gemäß einer Ausführungsform ist das mindestens eine Rückhalteelement als elastisch verformbarer Rastarm (Federzunge mit endseitiger Rastnase) ausgebildet, welcher eine Bewegung einer Flanke entgegen und/oder in der Einführungsrichtung der Flanke in die Sicherungshülse verhindert, indem er in ein entsprechendes Langloch in der Flanke einrastet. Hierbei entspricht die radiale Erstreckung des mindestens einen Rastarms bzw. dessen Rastnase in den Innenumfang der Sicherungshülse maximal der radialen Erstreckung des Anschlagsrings/Axialanschlags in den Innenumfang der Sicherungshülse. Hierdurch wird eine Verengung des zwischen den Flanken gebildeten Durchgangs durch den mindestens einen Rastarm verhindert.

In einer bevorzugten Ausführungsform ist die Sicherungshülse aus zwei Halbhülsen zusammengesetzt, die über die Anzahl an Nutensteinen an der Innenseite der Sicherungshülse und eine entsprechende Anzahl an korrespondierenden Gegensteinen an der Außenseite der Sicherungshülse nach dem Druckknopfprinzip fest miteinander verbunden sind. Alternativ kann die Sicherungshülse auch einstückig gefertigt sein oder die Halbhülsen anderweitig verbunden sein. Die Sicherungshülse kann aus Metall oder Kunststoff oder einem anderen vorteilhaften Werkstoff gefertigt sein.

Um die Stabilität der Sicherungshülse zu verbessern, kann die Sicherungshülse zusätzlich an ihrer Außenseite mehrere entlang der Längsachse der Sicherungshülse verlaufende Versteifungsrillen aufweisen.

### Figurenbeschreibung

Weitere Merkmale und Vorteile der vorliegenden Erfindung ergeben sich aus der folgenden Beschreibung bevorzugter Ausführungsformen mit Bezug auf die Figuren 1 bis 9. Hierbei zeigt:
Fig. 1 eine Seitenansicht einer erfindungsgemäßen Sicherungshülse,
Fig. 2 eine Seitenansicht einer erfindungsgemäßen Sicherungshülse von einem anderen Blickpunkt als in Fig.1,
Fig. 3 eine Stirnansicht einer erfindungsgemäßen Sicherungshülse,
Fig. 4 eine seitliche Draufsicht einer erfindungsgemäßen Sicherungshülse,
Fig. 5 eine Pedikelschraube mit verlängerten Flanken, auf die eine erfindungsgemäße Sicherungshülse aufgesetzt ist,
Fig. 6 eine Detailansicht einer auf eine Pedikelschraube mit verlängerten Flanken aufgesetzten erfindungsgemäßen Sicherungshülse,
Fig. 7 eine Detailansicht einer auf eine Pedikelschraube mit verlängerten Flanken aufgesetzten erfindungsgemäßen Sicherungshülse von einem anderen Blickpunkt als in Fig. 6,
Fig. 8 eine Detailansicht der Flanken einer Pedikelschraube, auf die eine erfindungsgemäße Sicherungshülse aufsetzbar ist,
Fig. 9 eine Ausrichtung mehrerer Pedikelschrauben mit verlängerten Flanken und auf die Flanken aufgesetzten erfindungsgemäßen Sicherungshülsen zueinander durch einen Korrekturstab.

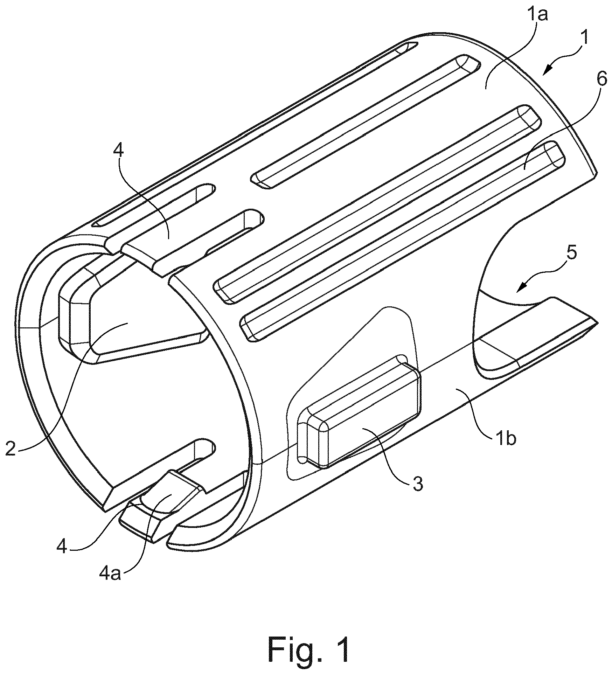

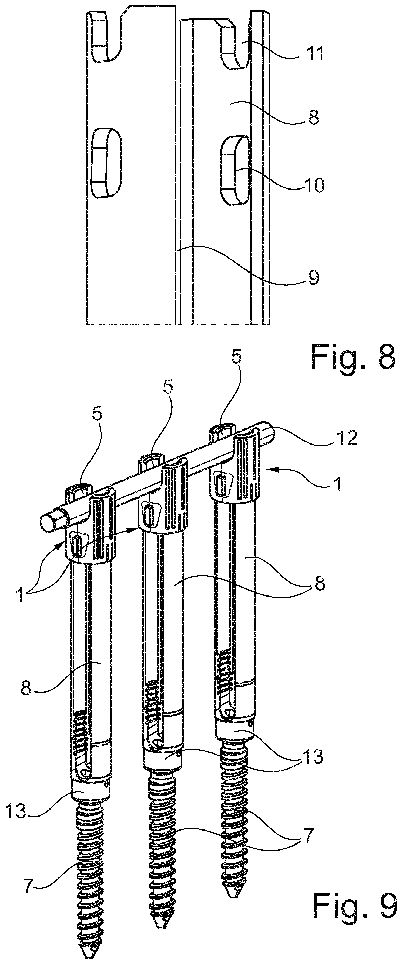

Wie in Fig. 1 gezeigt, ist die Sicherungshülse 1 aus zwei identischen Halbhülsen 1a, 1b zusammengesetzt, die über zwei axial konische Nutensteine 2 an der Innenseite der Sicherungshülse 1 und zwei entsprechende Gegensteine 3 an der Außenseite der Sicherungshülse 1 miteinander nach dem Druckknopfprinzip verbunden sind. Hierbei liegen sich jeweils ein Nutenstein 2 und ein Gegenstein 3 an der Innen- bzw. Außenseite der Sicherungshülse 1 gegenüber. Die beiden konischen Nutensteine an der Innenseite der Sicherungshülse 1 verjüngen sich in Richtung eines ersten Endes der Sicherungshülse 1 entgegen der Einführungsrichtung der Flanken einer allgemein bekannten Pedikelschraube gemäß dem eingangs genannten Stand der Technik in die Sicherungshülse 1. Zum Verhindern eines Abstreifens der Sicherungshülse 1 von den Flanken der Pedikelschraube entgegen und/oder in der Einführungsrichtung der Flanken der Pedikelschraube in die Sicherungshülse 1 weist die Sicherungshülse 1 an ihrem ersten Ende zwei elastisch verformbare sich axial erstreckende Rastarme oder Federzungen 4 auf. Jeder Rastarm 4 weist einen Vorsprung/ eine Rastnase 4a auf, welcher nach innen in den Innenumfang der Sicherungshülse 1 hervorsteht und in Richtung des ersten Endes der Sicherungshülse 1 keilförmig abgeflacht ist. An einem dem ersten Ende der Sicherungshülse 1 entgegengesetzten zweiten Ende weist die Sicherungshülse 1 zwei axial sich erstreckende Ausrichtenuten 5 auf (nur eine sichtbar in Fig. 1), die jeweils mit einem der einander diametral gegenüberliegenden konischen Nutensteine 2 axial fluchten und in die ein Korrekturstab (siehe Fig. 9) zum Ausrichten mehrerer Sicherungshülsen 1 zueinander quer zur Hülse 1 eingelegt werden kann. An ihrer Außenseite weist die Sicherungshülse 1 zusätzlich mehrere axiale Versteifungsrillen 6 auf.

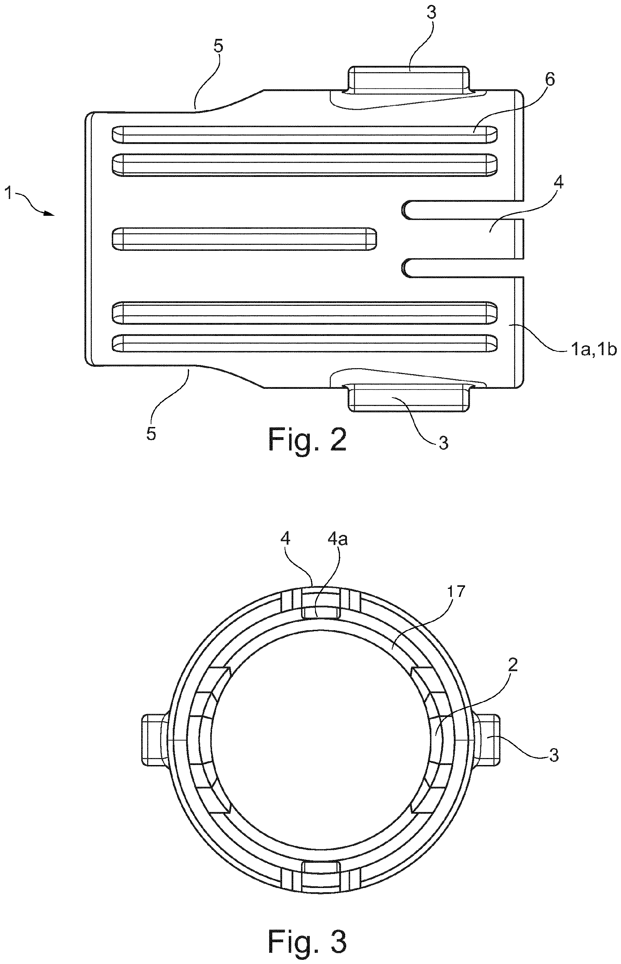

Fig. 2 zeigt die Sicherungshülse aus Fig. 1 in Seitenansicht von einem anderen Blickpunkt aus gesehen als in Fig. 1. In dieser Darstellung ist nur eine der beiden Halbhülsen 1a bzw. 1b sichtbar. Dieselben Bezugszeichen beziehen sich hierbei auf dieselben Bauteile. Wie hieraus zu entnehmen ist, wird die Federzunge/der Rastarm 4 durch zwei umfangsbeabstandete Axialschlitze gebildet, die in etwa auf 1/3 Hülsenlänge enden.

Fig. 3 zeigt eine Querschnittsansicht /Stirnansicht der erfindungsgemäßen Sicherungshülse 1, wobei das erste Ende der Sicherungshülse 1 dem Betrachter zugewandt ist. Dieselben Bezugszeichen beziehen sich hierbei auf dieselben Bauteile wie in den vorhergehenden Figuren. In dieser Ansicht ist auch ein ringförmiger Anschlagsring 17 zu erkennen, der entlang der gesamten inneren Umfangslinie der Sicherungshülse 1 ausgebildet ist und in den Innenumfang der Sicherungshülse 1 radial hervorragt. Weiterhin ist zu erkennen, dass die Nutensteine 2 und Rastarme 4 in radial nach innen gerichteter Richtung nicht in den durch den Anschlagsring 17 definierten Innenumfang der Sicherungshülse vorragen. Dadurch wird der Innendurchmesser der Sicherungshülse 1 und somit der instrumentenzugängliche Durchgang zwischen den Flanken der Pedikelschraube nicht verengt.

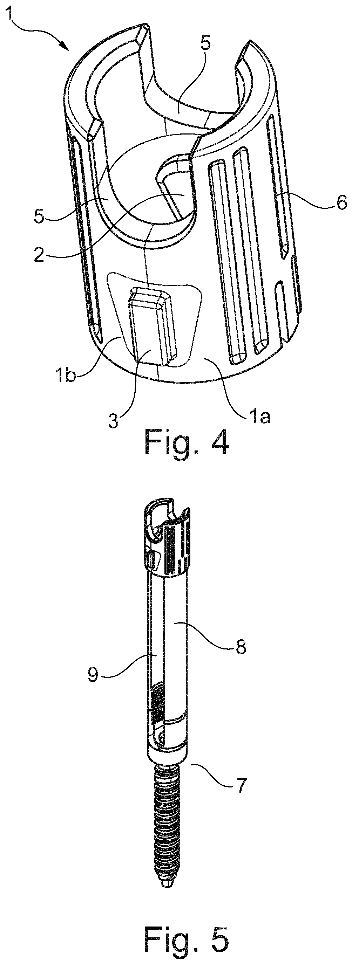

In Fig. 4 sind die beiden Ausrichtenuten 5 am zweiten Ende der Sicherungshülse 1 klar erkennbar. Jede dieser Ausrichtenuten 5 fluchtet axial mit jeweils einem Nutenstein 2.

Fig. 5 zeigt eine Pedikelschraube 7 mit verlängerten Flanken 8, auf die eine erfindungsgemäße Sicherungshülse 1 aufgesetzt ist. Die beiden verlängerten Flanken 8 der Pedikelschraube 7 bilden zwischen sich den instrumentenzugänglichen Durchgang 9. Durch die Nutensteine 2 der Sicherungshülse 1 werden die Flanken 8 in einem maximalen Abstand zueinender gehalten, um einen maximalen Durchmesser des Durchgangs 9 und somit ein möglichst einfaches Einführen von Instrumenten in den Durchgang 9 zu erreichen.

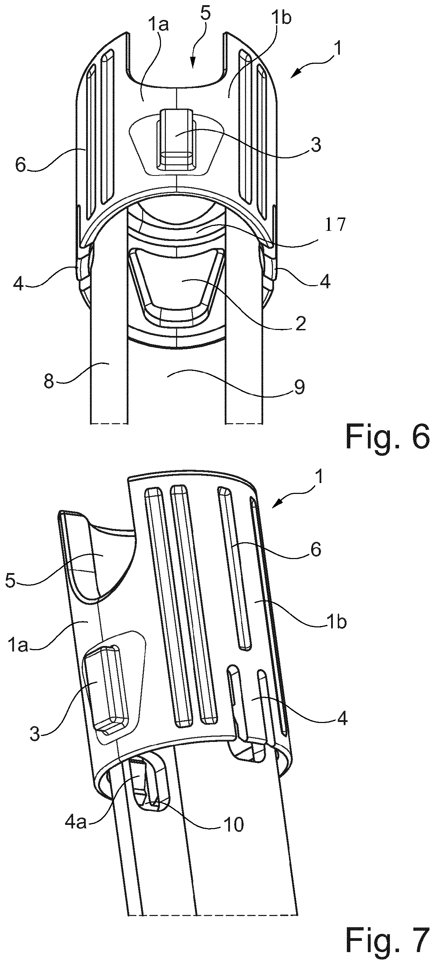

In Fig. 6 ist in Detailansicht gezeigt, wie die Flanken 8 in die Sicherungshülse eingelegt sind. Die konischen Nutensteine 2 sind zwischen die Flanken 8 der Pedikelschraube spreizend eingelegt und halten somit den Durchgang 9 zwischen den Flanken 8 offen. Die Flanken 8 liegen hierbei an der Sicherungshülse 1 mantelseitig an und verlaufen parallel zueinander. Der Anschlagsring 17 liegt auf den oberen Kanten der Flanken 8 jeweils auf und definiert die Aufnahmetiefe der Flanken 8 in die Sicherungshülse 1. Der Anschlagsring 17 steht hierbei nicht in den durch die Flanken 8 definierten Durchgang 9 hervor.

In Fig. 7 ist in Detailansicht gezeigt, wie die Rastarme 4 der Sicherungshülse 1 in jeweils ein Langloch 10 in jeweils einer der Flanken 8 eingreifen und somit ein ungewolltes Abstreifen der Sicherungshülse von den Flanken 8 verhindern. Die Rastarme 4 sind elastisch in Radialrichtung verformbar und weisen an ihrer der Innenseite der Sicherungshülse Zugewandten Seite jeweils einen Vorsprung 4a auf. Werden Flanken 8 in die Sicherungshülse 1 eingeführt, so werden die Rastarme 4 mit den Vorsprüngen 4a elastisch nach außen gedrückt, bis die Rastarme 4 jeweils auf ein Langloch 10 jeweils einer Flanke 8 treffen und in dieses einrasten. Wenn die Rastarme 4 in die Langlöcher 4a eingerastet sind, so verhindert der jeweils in ein Langloch 10 radial hineinragende Vorsprung 4a eines jeden Rastarms 4 eine Bewegung der Sicherungshülse 1 entgegen der Einführungsrichtung der Flanken 8. Durch das Zusammenspiel zwischen dem Anschlagsring 17, welcher die Position der Flanken 8 in der Sicherungshülse 1 in Einführungsrichtung definiert, den Rastarmen 4, welche die Position der Flanken 8 in der Sicherungshülse 1 entgegen der Einführungsrichtung definieren, und der Nutensteine, welche die Position der Flanken 8 in Umfangsrichtung der Sicherungshülse 1 definieren und eine Rotation der Flanken 8 in Umfangsrichtung der Sicherungshülse 1 verhindern, läßt sich die Position der Flanken 8 in der Sicherungshülse 1 in allen Richtungen fest definieren. Durch die besondere Ausgestaltung der Sicherungshülse 1 entstehen hierbei keinerlei Hinterschneidungen oder tote Winkel, sodass die Sicherungshülse 1 einfach zu reinigen ist.

Fig. 8 ist eine Detailansicht zweier Flanken 8 mit jeweils einem Langloch 10 zur Aufnahme eines Rastarms 4. Weiterhin weisen die Flanken 8 an ihrem der Pedikelschraube abgewandten Ende jeweils eine Nut 11 auf, die mit dem Langloch 10 der jeweiligen Flanke 8 fluchtet.

Wie in Fig. 9 gezeigt, erfüllt die Sicherungshülse 1 neben ihrer Funktion des Sicherns der verlängerten Flanken 8 einer Pedikelschraube 7 auch zusätzlich eine Funktion des Ausrichtens der Flanken 8 mehrerer Pedikelschrauben 7 zueinander. Hierfür wird ein Korrekturstab 12 in die Ausrichtenuten 5 mehrerer Sicherungshülsen 1, welche jeweils auf zwei Flanken 8 jeweils einer Pedikelschraube 7 aufgesetzt sind, eingelegt. Der Korrekturstab ist somit von den Tulpenköpfen 13 der Pedikelschrauben 7 um den Betrag der Länge der Flanken 8 beabstandet. Somit kann auch die Ausrichtung mehrerer Pedikelschrauben 7 zueinander minimalinvasiv und mit minimaler Gewebeschädigung durchgeführt werden.

## Patentansprüche

1. Sicherungshülse (1) für eine Pedikelschraube (7), mit:
einer Anzahl an Nutensteinen (2) an der Innenseite der Sicherungshülse (1), die dafür angepasst sind, um zwischen die Flanken (8) der Pedikelschraube (7) zum Definieren eines festen Abstands zwischen den Flanken (8) eingeführt zu werden, **dadurch gekennzeichnet, dass** sich mindestens einer der Nutensteine (2) konisch entgegen der Einführungsrichtung der Flanken (8) der Pedikelschraube (7) in die Sicherungshülse (1) verjüngt.

2. Sicherungshülse (1) nach Anspruch 1, mit weiterhin:
einem Anschlagsring oder Anschlagsvorsprung (17) an der Innenseite der Sicherungshülse (1), welcher dafür angepasst ist, um auf der axialen Oberkante der Flanken (8) aufzuliegen und die Aufnahmetiefe der Flanken (8) in die Sicherungshülse (1) zu begrenzen.

3. Sicherungshülse (1) nach Anspruch 2, **dadurch gekennzeichnet, dass** die radiale Erstreckung des Anschlagsrings oder Anschlagsvorsprungs (17) in den Innenumfang der Sicherungshülse (1) von dem Innenumfang des durch die Flanken (8) der Pedikelschraube (7) definierten Durchgangs (9) begrenzt ist.

4. Sicherungshülse (1) nach einem der vorstehenden Ansprüche, **dadurch gekennzeichnet, dass** die radiale Erstreckung der Anzahl an Nutensteinen (2) in den Innenumfang der Sicherungshülse (1) maximal der radialen Erstreckung des Anschlagsrings oder Anschlagsvorsprungs (17) in den Innenumfang der Sicherungshülse (1) entspricht.

5. Sicherungshülse (1) nach einem der vorstehenden Ansprüche, **gekennzeichnet durch** mindestens eine Ausrichtenut (5) an dem der Pedikelschraube abgewandten Ende der Sicherungshülse (1) zur Aufnahme eines Stabes (12) zur Ausrichtung der Flanken (8) mehrerer Pedikelschrauben (7) zueinander.

6. Sicherungshülse (1) nach Anspruch 5, **dadurch gekennzeichnet, dass** die mindestens eine Ausrichtenut (5) mit mindestens einem der Nutensteine (2) axial fluchtet.

7. Sicherungshülse (1) nach einem der vorstehenden Ansprüche, **gekennzeichnet durch** mindestens ein Rückhaltelement an dem der Pedikelschraube (7) zugewandten Ende der Sicherungshülse (1) zur Verhinderung eines unerwünschten Abstreifens der Sicherungshülse (1) von den Flanken (8) entgegen der Einführungsrichtung der Flanken (8) in die Sicherungshülse (1).

8. Sicherungshülse (1) nach Anspruch 7, **dadurch gekennzeichnet, dass** das mindestens eine Rückhalteelement als elastisch verformbarer Rastarm (4) ausgebildet ist, welcher eine Bewegung einer Flanke (8) entgegen der Einführungsrichtung der Flanke (8) in die Sicherungshülse (1) verhindert, indem er in ein entsprechendes Langloch (10) in der Flanke (8) einrastet.

9. Sicherungshülse (1) nach einem der vorstehenden Ansprüche, **dadurch gekennzeichnet, dass** die Sicherungshülse (1) aus zwei Halbhülsen (1a,1b) besteht, die über die Anzahl an Nutensteinen (2) an der Innenseite der Sicherungshülse (1) und mindestens eine Anzahl an korrespondierenden Gegensteinen (3) an der Außenseite der Sicherungshülse (1) nach dem Druckknopfprinzip fest miteinander verbunden sind.

10. Sicherungshülse (1) nach einem der Ansprüche 1 bis 8, **dadurch gekennzeichnet, dass** die Sicherungshülse (1) einstückig gefertigt ist.

11. Wirbelrepositionssystem mit zumindest einer Pedikelschraube (7) bestehend aus einem Schraubenschaft und einer Tulpe mit verlängerten Tulpenflanken (8), **gekennzeichnet durch**
eine Sicherungshülse (1) gemäß einem der Ansprüche 1-10.

## Claims

1. A locking sleeve (1) for a pedicle screw (7), comprising:
a number of groove blocks or slotnuts (2) on the inner side of the locking sleeve (1), which are adapted to be inserted between the legs (8) of the pedicle screw (7) for defining a fixed distance between the legs (8), **characterized in that** at least one of the groove blocks or slotnuts (2) tapers in conical fashion contrary to the direction of inserting the legs (8) of the pedicle screw (7) into the locking sleeve (1).

2. The locking sleeve (1) according to claim 1, further comprising:
a stop ring or stop protrusion (17) provided on the inner side of the locking sleeve (1) and adapted to rest on the axial upper edge of the legs (8) and to limit the accommodation depth of the legs (8) into the locking sleeve (1).

3. The locking sleeve (1) according to claim 2, **characterized in that** the radial extension of the stop ring or stop protrusion (17) into the inner circumference of the locking sleeve (1) is limited by the inner circumference of a passage (9) defined by the legs (8) of the pedicle screw (7).

4. The locking sleeve (1) according to any of the preceding claims, **characterized in that** the radial extension of the number of groove blocks or slotnuts (2) into the inner circumference of the locking sleeve (1) corresponds at the most to the radial extension of the stop ring or stop protrusion (17) into the inner circumference of the locking sleeve (1).

5. The locking sleeve (1) according to any of the preceding claims, **characterized by** at least one alignment groove (5) which is provided on the end of the locking sleeve (1) facing away from the pedicle screw and serves for receiving a rod (12) for aligning the legs (8) of several pedicle screws (7) with respect to one another.

6. The locking sleeve (1) according to claim 5, **characterized in that** the at least one alignment groove (5) is axially aligned with at least one of the groove blocks or slotnuts (2).

7. The locking sleeve (1) according to any of the preceding claims, **characterized by** at least one restraining element on the end of the locking sleeve (1) facing the pedicle screw (7) for preventing an undesired stripping of the locking sleeve (1) from the legs (8) contrary to the direction of inserting the legs (8) into the locking sleeve (1).

8. The locking sleeve (1) according to claim 7, **characterized in that** the at least one restraining element is formed as an elastically deformable latching arm (4) preventing a movement of a leg (8) contrary to the direction of inserting the leg (8) in the locking sleeve (1) by latching in place in a corresponding elongated hole (10) in the leg (8).

9. The locking sleeve (1) according to any of the preceding claims, **characterized in that** the locking sleeve (1) consists of two half sleeves (1a,1b) which are firmly connected to each other according to the press button principle by the number of groove blocks (2) on the inner side of the locking sleeve (1) and at least a number of corresponding mating blocks (3) on the outer side of the locking sleeve (1).

10. The locking sleeve (1) according to any of claims 1 to 8, **characterized in that** the locking sleeve (1) is manufactured in one piece.

11. A vertebral reposition system comprising at least one pedicle screw (7) consisting of a screw shaft and a tulip having extended tulip legs (8), **characterized by** a locking sleeve (1) according to any of claims 1 to 10.

## Revendications

1. Manchon de sécurité (1) pour une vis pédiculaire (7), avec :
un certain nombre de coulisseaux (2), au niveau du côté intérieur du manchon de sécurité (1), qui sont adaptés pour être introduits entre les flancs (8) de la vis pédiculaire (7) afin de définir une distance fixe entre les flancs (8), **caractérisé en ce qu'**au moins un des coulisseaux (2) se rétrécit en cône à l'opposé de la direction d'introduction des flancs (8) de la vis pédiculaire (7) dans le manchon de sécurité (1).

2. Manchon de sécurité (1) selon la revendication 1, avec en outre :
une bague de butée ou partie en saillie de butée (17), au niveau du côté intérieur du manchon de sécurité (1), qui est adaptée pour reposer sur l'arête supérieure axiale des flancs (8) et pour limiter la profondeur de logement des flancs (8) dans le manchon de sécurité (1).

3. Manchon de sécurité (1) selon la revendication 2, **caractérisé en ce que** l'extension radiale de la bague de butée ou partie en saillie de butée (17) dans la circonférence intérieure du manchon de sécurité (1) est limitée par la circonférence intérieure du passage (9) défini par les flancs (8) de la vis pédiculaire (7).

4. Manchon de sécurité (1) selon l'une quelconque des revendications précédentes, **caractérisé en ce que** l'extension radiale du certain nombre de coulisseaux (2) dans la circonférence intérieure du manchon de sécurité (1) correspond au maximum à l'extension radiale de la bague de butée ou partie en saillie de butée (17) dans la circonférence intérieure du manchon de sécurité (1).

5. Manchon de sécurité (1) selon l'une quelconque des revendications précédentes, **caractérisé par** au moins une rainure d'alignement (5) au niveau de l'extrémité, éloignée de la vis pédiculaire, du manchon de sécurité (1) afin de loger une barre (12) destinée à aligner les flancs (8) de plusieurs vis pédiculaires (7) les uns par rapport aux autres.

6. Manchon de sécurité (1) selon la revendication 5, **caractérisé en ce que** l'au moins une rainure d'alignement (5) est alignée de façon axiale avec au moins un des coulisseaux (2).

7. Manchon de sécurité (1) selon l'une quelconque des revendications précédentes, **caractérisé par** au moins un élément de retenue au niveau de l'extrémité, proche de la vis pédiculaire (7), du manchon de sécurité (1) afin d'empêcher un glissement non souhaité du manchon de sécurité (1) en retrait des flancs (8) à l'opposé de la direction d'introduction des flancs (8) dans le manchon de sécurité (1).

8. Manchon de sécurité (1) selon la revendication 7, **caractérisé en ce que** l'au moins un élément de retenue est réalisé comme un cran (4) déformable élastiquement qui empêche un déplacement d'un flanc (8) à l'opposé de la direction d'introduction du flanc (8) dans le manchon de sécurité (1) du fait qu'il s'enclenche dans un trou oblong (10) correspondant dans le flanc (8).

9. Manchon de sécurité (1) selon l'une quelconque des revendications précédentes, **caractérisé en ce que** le manchon de sécurité (1) est constitué de deux demi-manchons (la, 1b) qui sont reliés entre eux de manière fixe, selon le principe du bouton à pression, par l'intermédiaire du certain nombre de coulisseaux (2) au niveau du côté intérieur du manchon de sécurité (1) et d'au moins un nombre d'éléments complémentaires (3) correspondants au niveau du côté extérieur du manchon de sécurité (1).

10. Manchon de sécurité (1) selon l'une quelconque des revendications 1 à 8, **caractérisé en ce que** le manchon de sécurité (1) est fabriqué d'une seule pièce.

11. Système de repositionnement de vertèbre avec au moins une vis pédiculaire (7) constituée d'une tige de vis et d'une tulipe avec des flancs de tulipe (8) prolongés,
**caractérisé par**
un manchon de sécurité (1) selon l'une quelconque des revendications 1 à 10.
